# EUROPEAN PATENT SPECIFICATION

(11) **EP 3 083 071 B1**
(45) Date of publication and mention of the grant of the patent: **19.02.2020**
(21) Application number: 14820775.6
(22) Date of filing: 11.12.2014
(51) Int. Cl.: B05B 17/00

(54) **AN ASSEMBLY FOR USE IN LIQUID A DROPLET APPARATUS**
ANORDNUNG ZUR VERWENDUNG IN EINER FLÜSSIGKEITSTROPFENVORRICHTUNG
ENSEMBLE DESTINÉ À UNE UTILISATION DANS UN APPAREIL À GOUTTELETTES LIQUIDES

(30) Priority: 19.12.2013 EP 13198614
(43) Date of publication of application: 26.10.2016
(73) Proprietor: Koninklijke Philips N.V., 5656 AE Eindhoven (NL)
(72) Inventor: VON HOLLEN, Dirk Ernest, 5656 AE Eindhoven (NL)
(74) Representative: Schudelaro, Antonius Adrianus Petrus
(86) International application number: PCT/EP2014/077300
(87) International publication number: WO 2015/091171

(56) References cited:
- WO-A1-2012/092163
- WO-A2-2012/156724
- DE-U1-202012 105 005
- US-A- 3 790 079

## Description

### Technical Field

The invention relates to an assembly for use in a liquid droplet apparatus for producing liquid droplets, for example for use in ultrasonic mesh aerosol nebulizer devices, and in particular to an assembly such as a mesh sub-assembly.

### Background

Aerosol generators are used in various industries to produce fine droplets. For example, nebulisers deliver pharmaceuticals in droplet form, for example for inhalation.

There are many known nebuliser designs, which include human and spring powered devices. Much recent research has been directed to the use of electrically powered nebulizers, for example jet nebulisers (also called atomizers), which force a gas through a liquid containing the medicine, ultrasonic wave nebulisers, in which a piezoelectric element vibrates a column of liquid to produce a vapour mist, and vibrating mesh technology, in which an aperture plate is vibrated against the surface of a liquid reservoir, or has a supply of liquid supplied directly to the aperture plate. The aperture plate may comprise a mesh, a screen, a membrane, a machined metal plate, a polymer or the like, having many tiny openings or micro nozzles. When the aperture plate is vibrated (usually by a piezoelectric element), fine droplets are dispensed.

Nebulizers of this type comprise a mesh sub-assembly, often in the form of a ring, in which an aperture plate (such as a screen, mesh, membrane, machine metal plate, polymer or the like, as mentioned above) is coupled to a vibrating source (such as a piezoelectric element).

Previous ring style mesh aerosol systems are described with a surface island and a three dimensional structure that protrudes from a mounting plate, or as a single piece structure in which the mesh is permanently attached to the vibrating structure. Ring style mesh aerosol arrangements of this type are formed with the mesh being attached to the external surface or top of the island.

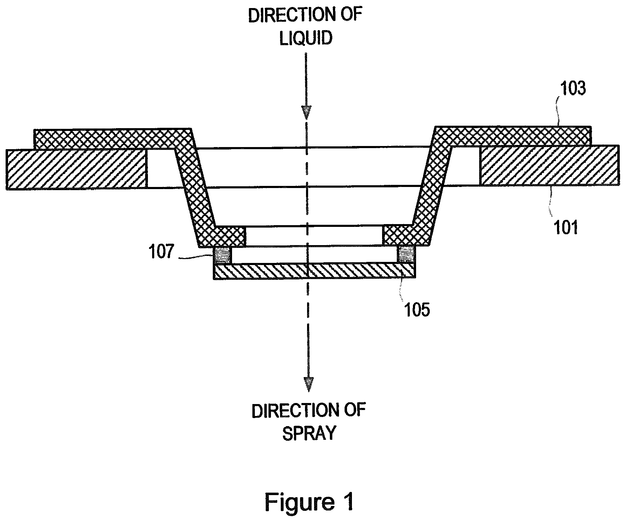

Figure 1 shows a mesh sub-assembly in which a aperture plate 105 is coupled to a vibrating platform 103, using as a non-limitative example an adhesive 107. The vibrating platform 103 couples the aperture plate 105 with a vibrating element 101 (such as a piezoelectric ring element). The vibrating platform 103 acts to transmit vibrations received from the vibrating element 101 to the aperture plate 105. The arrow shows the direction of spray during use, based on liquid being applied to the aperture plate 105 is a corresponding direction. During use, contraction of the vibrating element 101 in an inward radial direction causes the angled structure to become less angled or less sloped, i.e. more orthogonal to a plane in which the vibrating element 101 lies, thus causing the aperture plate 105 to move in a forward direction, that is towards the bottom of the page in the Figure, or in the direction of spray as shown by the arrow in Figure 1.

As can be seen from Figure 1, in such an arrangement the aperture plate 105 protrudes from the assembly. This has a disadvantage of requiring additional space. Furthermore, the aperture plate 105 is susceptible to being inadvertently dislodged or removed.

Patent application DE202012105055 discloses a mesh assembly comprising a vibrating platform having a first annular portion connected to a vibrating element and a second annular portion connected to the mesh, wherein the mesh lies in a cavity defined by the second annular portion.

Patent application US 3,790,079 discloses an apparatus for generating monodisperse aerosol. A disc is held within a circular recess of a plate. The vibrating platform has a sidewall that is surrounded by an annular means operable to vibrate.

### Summary

It is an aim of the present invention to provide an apparatus or method which obviates or reduces at least one or more of the disadvantages mentioned above. According to a first aspect of the present invention there is provided an assembly for use in a liquid droplet apparatus. The assembly comprises a vibrating element, an aperture plate and a vibrating platform. The vibrating platform is positioned between the vibrating element and the aperture plate, for conveying vibrations from the vibrating element to the aperture plate. The vibrating platform is structured such that the aperture plate is located within a cavity formed in the vibrating platform.

This has the advantage of protecting the aperture plate from accidental dislodgement from the vibrating platform. This structure also has the advantage of reducing the overall height of the assembly.

### Brief description of the drawings

For a better understanding of examples of the present invention, and to show more clearly how the examples may be carried into effect, reference will now be made, by way of example only, to the following drawings in which:
Figure 1 shows a known sub-assembly;
Figure 2 shows an embodiment of the present invention;

### Detailed description

Preliminary, it should be clearly understood that in the meaning of the invention the terms "vibrating element" and "vibrating platform" equally designate or be construed as an element and a platform that are configured to vibrate, respectively. It should further be clear that in the meaning of the invention any vibration related to this element of platform should occur during use only.

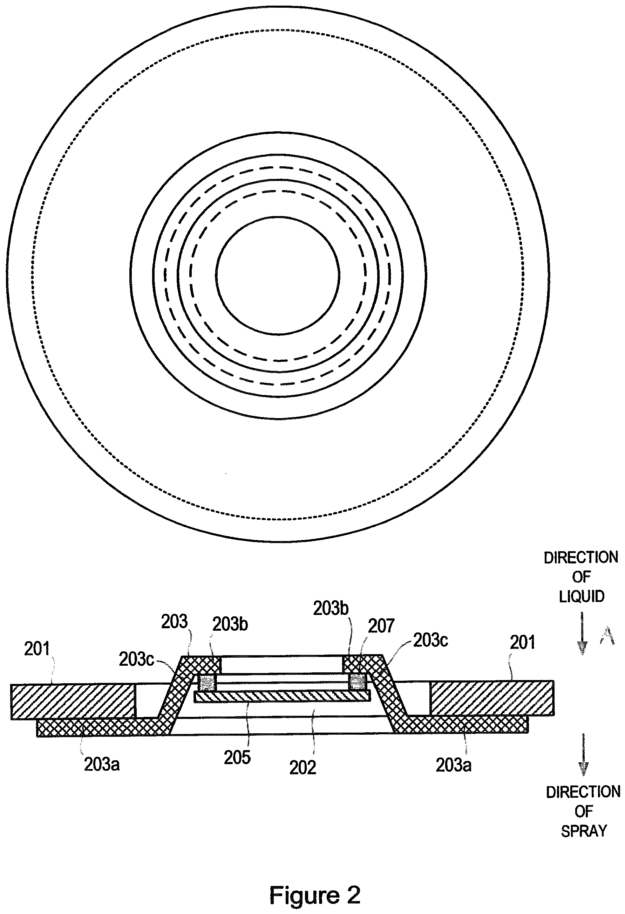

Figure 2 shows a cross-sectional view and a plan view of an assembly according to an embodiment of the present invention, for use in a liquid droplet apparatus.

The assembly comprises a vibrating element 201, an aperture plate 205 and a vibrating platform 203. The vibrating platform 203 is positioned between the vibrating element 201 and the aperture plate 205 for conveying vibrations from the vibrating element 201 to the aperture plate 205. The vibrating platform 203 is structured such that the aperture plate 205 is located within a cavity 202 formed in the vibrating platform 203.

In the example of Figure 2 the vibrating element 201 takes the form of an annular vibrating element, for example an annular piezoelectric device, also known as a piezoelectric ring, wherein during use the annular piezoelectric device is radially expandable and contractable upon actuation thereof, i.e. vibrates in a radial direction. The annular piezoelectric device also vibrates in an axial direction.

The aperture plate 205 may comprise, for example, a mesh, a screen, a membrane, a machined metal plate (for example involving electroforming and laser ablation), a polymer or the like, having many tiny openings or micro nozzles. For example, the aperture plate 205 may be flat as shown, or domed in shape.

The embodiment of Figure 2 has the advantage of protecting the aperture plate 205 from accidental dislodgement from the vibrating platform (by the manner in which it is located within a cavity 202 formed by the vibrating platform 203).

In the example of Figure 2 the vibrating platform 203, having the aperture plate 205 arranged within a cavity 202 thereof, is structured such that a sidewall 203c of the vibrating platform (coupling a first annular portion 203a and a second annular portion 203b) protrudes at least partly through the opening in the annular vibrating element 201.

In the example of Figure 2 the vibrating platform 203, having the aperture plate 205 arranged within a cavity 202 thereof, is structured such that a sidewall 203c of the vibrating platform (coupling a first annular portion 203a and a second annular portion 203b) protrudes at least partly through the opening in the annular vibrating element 201. This has the further advantage of reducing the overall height of the assembly.

The assembly of Figure 2 is therefore effectively inverted compared to the structure of Figure 1, with the vibrating platform 203 being attached, in this example, to the vibrating element 201 on a side corresponding to the direction of spray. In such an example, movement of the aperture plate in a direction of the spray (referred to herein as a "forward" direction) is caused by expansion of the vibrating element in an outwardly radial direction, rather than by contraction in an inwardly radial direction as required in Figure 1.

It is noted that, if desired, the direction of spray can be reversed in Figure 2 by changing the direction in which liquid is applied to the aperture plate, for example such that the direction of liquid and direction of spray are opposite to that shown in Figure 2. In such an embodiment a "forward" movement of the aperture plate 205 is obtained by contraction of the vibrating element 201 in an inwardly radial direction, similar to that of Figure 1.

In Figures 2, the first annular portion 203a of the vibrating platform can be fixedly coupled to an annular portion of the vibrating element 201, and the second annular portion 203b can be fixedly coupled to the aperture plate 205. For example, the aperture plate 205 may be coupled to the vibrating platform 203 using adhesive 207, and the first portion 203a of the vibrating platform may be fixedly connected to the vibrating element 201 using an adhesive (not shown).

The embodiments of the present invention allow the aperture plate component 205 to vibrate in direction "A" when the annular piezoelectric element expands outward in a radial direction, in contrast to traditional mesh ring style aerosol generators which rely on the inward radial movement to provide a compression force to the mesh in order to vibrate in direction "A", which can also be termed a "forward" direction. The forward direction action allows fluid to be transmitted from the back side surface to the micro nozzles to the front surface as a spray. The embodiments of the present invention can therefore operate effectively in reverse compared to a conventional device, i.e. radial expansion rather than contraction of the annular piezoelectric device causes a forward movement of the aperture plate 205 in direction "A". This has the advantage of requiring less power to deliver a higher output rate of aerosol.

The inward cone or cavity defined by the embodiments of the invention provides a means of a built in barrier to prevent accidental removal of the aperture plate 205, for example protecting the aperture plate from being inadvertently removed from the adhesive which affixes the aperture plate to the vibrating platform 203.

The vibrating platform 203 may comprise at least one flexible joint (for example a joint which is non-stressed during manufacture), for allowing additional movement of the vibrating platform in response to movement of the vibrating element in a radial direction. This allows for additional movement (amplitude)", in a similar manner as a speaker when more power is added. Thus, having a flexible joint enables a greater amplitude of movement to be obtained for a given power that is applied to the piezoelectric device. This has the advantage of being able to increase the amplitude of movement without having to necessarily increase the power applied to the piezoelectric device, which can have power saving advantages, in addition to preventing damage to the piezoelectric device.

The geometry related to the inward facing structure that expands in the outward radial motion of a ring style piezo vibrator, provides the advantages of being able to move the aperture plate in response to radial movement of the vibrating element, and also protect the aperture plate within the cavity formed by the inward facing structure.

According to one embodiment the vibrating platform comprises a first surface for coupling to a vibrating element, and a second surface for coupling to an aperture plate, the plane of the first surface being separated from the plane of the second surface by sidewalls that couple the first and second surface.

The sidewalls define a cavity. The aperture plate is located within the cavity, on a side of the second surface which is inward facing to the cavity.

The embodiments of the invention can be applied to any form of respiratory drug delivery apparatus, including a nebulizer or atomizer. Applications include, for example, respiratory care or sleep assistance - nebulizer for delivery for home humidification via nasal cannula or mask.

The embodiments of the invention can be used to support efforts in home health care solutions as a part of a liquid nebulizer drug delivery system, or a humidification system for critical or home ventilation or sleep or nasal cannula via oxygen.

The embodiments of the invention provide an improved ultrasonic mesh aerosol nebulizer, in which the mesh sub-assembly is equal or lower than a piezo ring assembly.

It is noted that, in any of the embodiments described above, the aperture plate can take other shapes or forms, for example in addition to the flat or domed shapes described herein.

According to one embodiment there is provided an assembly for use in a liquid droplet apparatus. The assembly comprises a vibrating element, an aperture plate and a vibrating platform for coupling the vibrating element and the aperture plate. The vibrating platform is structured such that the aperture plate is positioned within a conical structure formed by the vibrating platform.

According to another embodiment there is provided an assembly for use in a liquid droplet apparatus. The assembly comprises a vibrating element, an aperture plate and a vibrating platform for coupling the vibrating element and the aperture plate. The vibrating platform is formed from an inverted structure, such that the aperture plate is positioned within a conical structure formed by the vibrating platform.

The sidewalls of the vibrating platform may be angled with respect to a plane of the vibrating element, and wherein expansion of the vibrating element in an outwardly radial direction causes the sidewalls of the vibrating platform to become more orthogonal with respect to the plane of the vibrating element, thereby causing the aperture plate to move in a forward direction.

The vibrating platform may be circular in shape, and comprise a first surface for coupling to the vibrating element, and a second surface for coupling to the aperture plate, the plane of the first surface being separated from the plane of the second surface by a sidewall that couples the first and second surfaces.

The aperture plate may be located within the cavity, on a side of the second surface which is inward facing to the cavity.

The sidewall may be angled with respect to the plane of the vibrating element. In such an embodiment, the angle of the sidewall is adjusted through contraction and expansion of the vibrating element in a radial direction in the plane of the vibrating element.

The vibrating platform may comprise a joint which is non-stressed during manufacture, for allowing additional movement of the vibrating platform in response to movement of the vibrating element in a radial direction.

Although the examples show the diameter of the annular vibrating element 201 being larger than the diameter of the first annular portion 203a of the vibrating platform 203, it is noted that the diameter of the annular vibrating element 201 may be smaller than, or equal to, the diameter of the first annular portion 203a of the vibrating platform 203, without departing from the scope of the invention as defined in the appended claims.

It should be noted that the above-mentioned embodiments illustrate rather than limit the invention, and that those skilled in the art will be able to design many alternative embodiments without departing from the scope of the appended claims. The word "comprising" does not exclude the presence of elements or steps other than those listed in a claim, "a" or "an" does not exclude a plurality, and a single processor or other unit may fulfil the functions of several units recited in the claims. Any reference signs in the claims shall not be construed so as to limit their scope.

## Claims

1. An assembly for use in a liquid droplet apparatus, the assembly comprising:
a vibrating element (201);
an aperture plate (205); and
a vibrating platform (203);
wherein the vibrating platform is positioned between the vibrating element and the aperture plate for conveying vibrations from the vibrating element to the aperture plate, wherein the vibrating platform comprises: a first annular portion (203a) for coupling to the vibrating element (201); second annular portion (203b) for coupling to the aperture plate (205); and a sidewall (203c) to define a cavity (202);
wherein the first annular portion (203a) lies in a first plane and the second annular portion (203b) lies in a second plane, the second plane being separated from and substantially in parallel with the first plane, and separated from and substantially in parallel with a third plane corresponding to a plane in which the vibrating element (201) lies,
wherein the sidewall protrudes at least partly through an aperture of the vibrating element,
and wherein the vibrating platform (203) is structured such that the aperture plate (205) is located within the cavity (202) defined by the sidewall;
**characterized in that** the sidewall is angled with respect to the plane of the vibrating element so that the angle of the sidewall is adjusted through contraction and expansion of the vibrating element in a radial direction in the plane of the vibrating element.

2. An assembly as claimed in claim 1, wherein the vibrating element (201) comprises an annular piezoelectric device, wherein during use the annular piezoelectric device is radially expandable and contractable upon actuation thereof.

3. An assembly as claimed in claim 1, wherein the sidewall (203c) is configured such that any expansion of the vibrating element (201) during use in a radially outward direction causes the sidewall (203c) of the vibrating platform (203) to become less orthogonal with respect to the first and second planes of the vibrating element, thereby causing the aperture plate (205) to move, during use, in a direction corresponding to a direction of spray.

4. An assembly as claimed in any one of claims 1 to 3, wherein the first annular portion (203a) of the vibrating platform is fixedly coupled to an annular portion of the vibrating element (201), and the second annular portion (203b) fixedly coupled to the aperture plate (205).

5. An assembly as claimed in any one of claims 1 to 4, wherein the vibrating platform (203) is configured such that the sidewall (203c) of the vibrating platform passes through an aperture in the vibrating element (201).

6. An assembly as claimed in claim 4 or 5, wherein the aperture plate (205) is located within the cavity (202) such that the aperture plate is coupled to a side of the second annular portion (203b) which is inward facing to the cavity (202).

7. An assembly as claimed in any one of the preceding claims, wherein the vibrating platform (203) is structured such that the aperture plate (205) is positioned within a cavity comprising a conical structure formed by the vibrating platform (203).

8. An assembly as claimed in any one of claims 1 to 6, wherein the vibrating platform (203) is formed from an inverted structure, such that the aperture plate (205) is positioned within a conical structure formed by the vibrating platform (203).

9. An assembly as claimed in any one of claims 1 to 9, wherein the first annular portion (203a) of the vibrating element is fixed to the annular piezoelectric device (201) on a side corresponding to a direction of flow of a fluid during use, and wherein the sidewall (203c) and second annular portion (203b) of the vibrating element project at least into the aperture of the annular piezoelectric device (201) in a direction opposite to the direction of flow.

10. An assembly as claimed in any one of the preceding claims, wherein the vibrating platform (203) comprises a joint which is non-stressed during manufacture, for allowing additional movement of the vibrating platform, during use, in response to movement of the vibrating element in a radial direction.

11. An assembly as claimed in any one of claims 1 to 9, wherein the vibrating platform (203) comprises at least one flexible joint (203d; 203e; 302f).

12. An assembly as claimed in any one of claims 1 to 11, wherein the vibrating platform (203) is structured such that one or more intersections between the first annular portion 203a, second annular portion 203b and sidewall 203c are curved.

## Patentansprüche

1. Anordnung zur Verwendung in einer Flüssigkeitstropfeneinrichtung, wobei die Anordnung umfasst:
- ein Schwingelement (201);
- eine Öffnungsplatte (205); und
- eine Schwingplattform (203);
wobei die Schwingplattform zwischen dem Schwingelement und der Öffnungsplatte positioniert ist, um Schwingungen von dem Schwingelement zu der Öffnungsplatte zu befördern, wobei die Schwingplattform Folgendes umfasst: einen ersten ringförmigen Abschnitt (203a) zum Koppeln mit dem Schwingelement (201); einen zweiten ringförmigen Abschnitt (203b) zum Koppeln mit der Öffnungsplatte (205); und eine Seitenwand (203c) zum Definieren eines Hohlraums (202);
wobei der erste ringförmige Abschnitt (203a) in einer ersten Ebene liegt und der zweite ringförmige Abschnitt (203b) in einer zweiten Ebene liegt, wobei die zweite Ebene von der ersten Ebene getrennt ist und im Wesentlichen parallel dazu ist, und von einer dritten Ebene, die einer Ebene entspricht, in der das Schwingelement (201) liegt, getrennt ist und im Wesentlichen parallel dazu ist,
wobei die Seitenwand mindestens teilweise durch eine Öffnung des Schwingelements hervorspringt,
und wobei die Schwingplattform (203) strukturiert ist, sodass sich die Öffnungsplatte (205) innerhalb des Hohlraums (202) befindet, der durch die Seitenwand definiert wird;
**dadurch gekennzeichnet, dass** die Seitenwand in Bezug auf die Ebene des Schwingelements abgewinkelt ist, sodass der Winkel der Seitenwand durch Zusammenziehen und Erweitern des Schwingelements in einer radialen Richtung in der Ebene des Schwingelements angepasst wird.

2. Anordnung nach Anspruch 1, wobei das Schwingelement (201) eine ringförmige piezoelektrische Vorrichtung umfasst, wobei die ringförmige piezoelektrische Vorrichtung während des Gebrauchs bei einer Betätigung davon radial erweiterbar und zusammenziehbar ist.

3. Anordnung nach Anspruch 1, wobei die Seitenwand (203c) konfiguriert ist, sodass eine Erweiterung des Schwingelements (201) während des Gebrauchs in eine radial nach außen führende Richtung bewirkt, dass die Seitenwand (203c) der Schwingplattform (203) in Bezug auf die erste und zweite Ebene des Schwingelements weniger orthogonal wird, wodurch bewirkt wird, dass sich die Öffnungsplatte (205) während des Gebrauchs in eine Richtung bewegt, die einer Sprührichtung entspricht.

4. Anordnung nach einem der Ansprüche 1 bis 3, wobei der erste ringförmige Abschnitt (203a) der Schwingplattform fest an den ringförmigen Abschnitt des Schwingelements (201) gekoppelt ist, und der zweite ringförmige Abschnitt (203b) fest an die Öffnungsplatte (205) gekoppelt ist.

5. Anordnung nach einem der Ansprüche 1 bis 4, wobei die Schwingplattform (203) konfiguriert ist, sodass die Seitenwand (203c) der Schwingplattform durch eine Öffnung in dem Schwingelement (201) hindurchverläuft.

6. Anordnung nach Anspruch 4 oder 5, wobei sich die Öffnungsplatte (205) innerhalb des Hohlraums (202) befindet, sodass die Öffnungsplatte an eine Seite des zweiten ringförmigen Abschnitts (203b) gekoppelt ist, der dem Hohlraum (202) nach innen gerichtet zugewandt ist.

7. Anordnung nach einem der vorstehenden Ansprüche, wobei die Schwingplattform (203) strukturiert ist, sodass die Öffnungsplatte (205) innerhalb eines Hohlraums positioniert ist, der eine konische Struktur umfasst, die durch die Schwingplattform (203) gebildet wird.

8. Anordnung nach einem der Ansprüche 1 bis 6, wobei die Schwingplattform (203) aus einer umgekehrten Struktur gebildet wird, sodass die Öffnungsplatte (205) innerhalb einer konischen Struktur positioniert ist, die durch die Schwingplattform (203) gebildet wird.

9. Anordnung nach einem der Ansprüche 1 bis 9, wobei der erste ringförmige Abschnitt (203a) des Schwingelements an der ringförmigen piezoelektrischen Vorrichtung (201) an einer Seite befestigt ist, die einer Strömungsrichtung eines Fluids während des Gebrauchs entspricht, und wobei die Seitenwand (203c) und der zweite ringförmige Abschnitt (203b) des Schwingelements mindestens in die Öffnung der ringförmigen piezoelektrischen Vorrichtung (201) in einer Richtung entgegengesetzt zu der Strömungsrichtung vorspringt.

10. Anordnung nach einem der vorstehenden Ansprüche, wobei die Schwingplattform (203) eine Verbindung umfasst, die während der Herstellung nicht beansprucht wird, um eine zusätzliche Bewegung der Schwingplattform während des Gebrauchs als Reaktion auf die Bewegung des Schwingelements in einer radialen Richtung zu erlauben.

11. Anordnung nach einem der Ansprüche 1 bis 9, wobei die Schwingplattform (203) mindestens eine flexible Verbindung (203d; 203e; 302f) umfasst.

12. Anordnung nach einem der Ansprüche 1 bis 11, wobei die Schwingplattform (203) strukturiert ist, sodass ein oder mehrere Schnittpunkte zwischen dem ersten ringförmigen Abschnitt 203a, zweiten ringförmigen Abschnitt 203b und Seitenwand 203c gekrümmt sind.

## Revendications

1. Ensemble destiné à une utilisation dans un appareil à gouttelettes liquides, l'ensemble comprenant :
un élément vibrant (201) ;
une plaque à orifice (205) ; et
une plate-forme vibrante (203) ;
dans lequel la plate-forme vibrante est placée entre l'élément vibrant et la plaque à orifice pour transmettre des vibrations de l'élément vibrant à la plaque à orifice, dans lequel la plate-forme vibrante comprend : une première partie annulaire (203a) destinée à être couplée à l'élément vibrant (201) ; une seconde partie annulaire (203b) destinée à être couplée à la plaque à orifice (205) ; et une paroi latérale (203c) pour définir une cavité (202) ;
dans lequel la première partie annulaire (203a) repose dans un premier plan et la seconde partie annulaire (203b) repose dans un deuxième plan, le deuxième plan étant séparé du premier plan et sensiblement parallèle à celui-ci, et séparé d'un troisième plan correspondant à un plan dans lequel l'élément vibrant (201) repose, et sensiblement parallèle à celui-ci,
dans lequel la paroi latérale fait saillie au moins en partie à travers un orifice de l'élément vibrant,
et dans lequel la plate-forme vibrante (203) est structurée de manière telle que la plaque à orifice (205) est située dans la cavité (202) définie par la paroi latérale ;
**caractérisé en ce que** la paroi latérale est inclinée par rapport au plan de l'élément vibrant de telle sorte que l'angle de la paroi latérale est ajusté par la contraction et la dilatation de l'élément vibrant dans une direction radiale dans le plan de l'élément vibrant.

2. Ensemble selon la revendication 1, dans lequel l'élément vibrant (201) comprend un dispositif piézoélectrique annulaire, dans lequel, lors de l'utilisation, le dispositif piézoélectrique annulaire est radialement dilatable et contractable lors de l'actionnement de celui-ci.

3. Ensemble selon la revendication 1, dans lequel la paroi latérale (203c) est configurée de telle sorte que toute dilatation de l'élément vibrant (201) lors de l'utilisation dans une direction radialement externe amène la paroi latérale (203c) de la plate-forme vibrante (203) à devenir moins perpendiculaire par rapport aux premier et deuxième plans de l'élément vibrant, provoquant ainsi le déplacement de la plaque à orifice (205), lors de l'utilisation, dans une direction correspondant à une direction de pulvérisation.

4. Ensemble selon l'une quelconque des revendications 1 à 3, dans lequel la première partie annulaire (203a) de la plate-forme vibrante est couplée à demeure à une partie annulaire de l'élément vibrant (201), et la seconde partie annulaire (203b) est couplée à demeure à la plaque à orifice (205).

5. Ensemble selon l'une quelconque des revendications 1 à 4, dans lequel la plate-forme vibrante (203) est configurée de telle sorte que la paroi latérale (203c) de la plate-forme vibrante traverse un orifice dans l'élément vibrant (201).

6. Ensemble selon la revendication 4 ou 5, dans lequel la plaque à orifice (205) est située dans la cavité (202) de telle sorte que la plaque à orifice est couplée à un côté de la seconde partie annulaire (203b) qui est orientée vers l'intérieur de la cavité (202).

7. Ensemble selon l'une quelconque des revendications précédentes, dans lequel la plate-forme vibrante (203) est structurée de telle sorte que la plaque à orifice (205) est placée dans une cavité comprenant une structure conique formée par la plate-forme vibrante (203).

8. Ensemble selon l'une quelconque des revendications 1 à 6, dans lequel la plate-forme vibrante (203) est formée à partir d'une structure inversée, de telle sorte que la plaque à orifice (205) est placée dans une structure conique formée par la plate-forme vibrante (203).

9. Ensemble selon l'une quelconque des revendications 1 à 9, dans lequel la première partie annulaire (203a) de l'élément vibrant est fixée au dispositif piézoélectrique annulaire (201) sur un côté correspondant à une direction de flux d'un fluide lors de l'utilisation, et dans lequel la paroi latérale (203c) et la seconde partie annulaire (203b) de l'élément vibrant font saillie au moins dans l'orifice du dispositif piézoélectrique annulaire (201) dans une direction opposée à la direction de flux.

10. Ensemble selon l'une quelconque des revendications précédentes, dans lequel la plate-forme vibrante (203) comprend un joint qui est non contraint pendant la fabrication, pour permettre un mouvement supplémentaire de la plate-forme vibrante, lors de l'utilisation, en réponse au mouvement de l'élément vibrant dans une direction radiale.

11. Ensemble selon l'une quelconque des revendications 1 à 9, dans lequel la plate-forme vibrante (203) comprend au moins un joint flexible (203d ; 203e ; 302f).

12. Ensemble selon l'une quelconque des revendications 1 à 11, dans lequel la plate-forme vibrante (203) est structurée de telle sorte qu'une ou plusieurs intersections entre la première partie annulaire 203a, la seconde partie annulaire 203b et la paroi latérale 203c sont incurvées.
